# EUROPEAN PATENT APPLICATION

(11) **EP 1 656 935 A1**
(43) Date of publication of application: **17.05.2006**
(21) Application number: 04026979.7
(22) Date of filing: 12.11.2004
(51) Int. Cl.: A61K 31/201, A61K 31/202, A61K 31/231, A61K 31/232, A61K 31/704, A61K 36/484, A61P 17/04

(54) **Use of physiologically active fatty acids for the treatment of pruritus**

(71) Applicant: Cognis IP Management GmbH, 40589 Düsseldorf (DE)
(72) Inventor: Prous, Santiago Rull, 08034 Barcelona (ES)
(74) Representative: Fabry, Bernd

(57) **Abstract**

Suggested is the use of physiologically active fatty acids, their salts and their esters, optionally in combination with extracts of *Glycyrrhiza glabra* or its active principles, for making a medicament for the treatment of pruritus.

## Description

### Object of the invention

The present invention relates to the area of pharmaceutical compositions and provides the use of a new active agent or composition for the treatment of pruritus (itching).

### State of the art

Pruritus, often also called "itching", is a peculiar tingling or uneasy irritation of the skin that causes a desire to scratch the affected area. Unfortunately, its mechanism has not been completely elucidated.

Disorder characterized by pruritus is distinguished into two types: itching skin disorder (for example, atopic dermatitis, urticaria, psoriasis, xeroderma, and trichophytia) and pruritus cutaneous which is not associated with skin disorder and provokes itching due to kidney dialysis and internal organ diseases [for example, diabetes, blood disease, cholestatic hepatitis (primary biliary liver cirrhosis) and kidney disease], hyperthyroidism and multiple sclerosis. In addition, the disease associated with severe itching includes diseases of cornea and conjunctiva, for example, allergic conjunctivitis. Recently, the number of patients with these diseases has rapidly increased and constitute a large problem in view of QOL (quality of life). Most itching diseases are common by the fact that a vicious circle is caused by injuries due to scratching. Histamine is known as a typical itch-producing substance and provokes itching in case it is added externally and internally isolated from mast cells.

As an anti-pruritus method, it is a generally known method to treat the primary disease while itch is controlled by an antihistamine agent or a suppressor of chemical mediator release. Besides, for an itch therapy it is also common, for example, to administer adrenal cortex hormone to suppress inflammation, or sedatives to suppress psychogenic causes, and to apply moisturizers for dry skin, and the like.

For example, **US 6,750,231** (Pfizer) suggests the administration of 4-arylpiperidine derivatives. **US 6,593,370** (Marruishi Pharmaceuticals) discloses the use of capsaicin for topical application in order to fight various types of painful cutaneous disorders and neural dysfunction, which includes itching. **US 2003/0125308** proposes topical compositions comprising acyl salicylates as the main active ingredient, however, from clinical studies it is known that the significance of aspirin for the treatment of pruritus is only about 50 %.

However, because of its side effects or insufficient performance, none of the various teachings from the state of the art is satisfying for the treatment of itching due to pruritic dermatitis. In addition, it has recently been reported that compounds other than histamine take part in itching due to atopic dermatitis. In many clinical cases, the antihistaminic agent and the antiallergic agent do not actually exert a remarkable effect on itching due to atopic dermatitis. In the treatment of pruritus cutaneous, the antihistamines or steroids are sometimes prescribed, however, they exert almost no effect, and thus no effective therapy does exist at present.

Since there are no satisfactory medicaments for the treatment of diseases associated with itching so far, the problem underlying the present invention has been to develop a medicament either for oral or topical administration, which
- effectively suppresses itching regardless of causative diseases from a clinical point of view,
- is based on natural sources, and
- does not cause unwanted side-effects.

### Description of the invention

A first object of the present invention claims the use of physiologically active fatty acids, their salts and their esters for preparing a medicament for the treatment of pruritus (itching).

A second object of the present invention is directed at active compositions comprising
(a) physiologically active fatty acids, their salts and their esters, and
(b) extracts of *Glycyrrhiza glabra* or its active principles
for preparing a medicament for the treatment of pruritus (itching).

Surprisingly, it has been found that physiologically active fatty acids, predominantly unsaturated fatty acids like conjugated linoleic acid (CLA) or omega-3 fatty acids, which are mainly derived from marine sources, their salts and esters, significantly reduce the desire to scratch the irritated area of the skin. In combination with extracts of *Glycyrrhiza glabra* in general, or its main active ingredient, glycyrrhizinic acid, even better effects have been found. In addition, while the fatty acids administered alone need some time to develop their benefits, the mixtures exhibit a higher activity. Finally, both the fatty acids and the mixtures are toxicologically and dermatologically safe.

### Physiologically active fatty acids, their salts and their esters

A common criterion for fatty acids with physiological activity, which represent component (a), is a fat chain having a sufficient number of carbon atoms providing a lipophilic behaviour that allows the molecule to pass through the gastrointestinal tract of the body, and a sufficient number of double bonds. Therefore, said fatty acids usually comprise 18 to 26 carbon atoms and 2 to 6 double bonds.

In a first embodiment of the present invention, conjugated linoleic acid (CLA) or its alkaline or alkaline earth salts and esters, preferably esters with lower aliphatic alcohols having 1 to 4 carbon atoms - or their glycerides, specially their triglycerides, come into account. Conjugated linoleic acid (CLA) represents a commercially available product which usually is obtained by base-catalysed isomerisation of sunflower oil or its respective alkyl esters and subsequent isomerisation in the presence of enzymes. CLA is an acronym used for positional and geometric isomers deriving from the essential fatty acid linoleic acid (LA, cis-9,cis-12-octadecadienoic acid, 18:2n-6). From a physiological point of view, the use of the *cis-*9,*trans-*11 isomer, according to the present invention, is of special importance having at least 30, preferably at least 50 and most preferably at least 80 % b.w. of said *cis*-9,*trans*-11 isomer, based on the total CLA content of the crude mixture. In addition, it has been found advantageous if the content of the *trans*-10,*cis*-12 isomer is at most 45, preferably at most 10 % b.w. and most preferably is less than 1 % b.w., and the sum of 8,10-, 11,13- and *trans,trans-*isomers in total is less than 1 % b.w. - again based on the total CLA content. Such products can be found in the market, for example, under the trademark Tonalin® CLA-80 (Cognis).

In a second embodiment, also so-called omega-3 fatty acids can come into account, which typically comprise 18 to 26, preferably 20 to 22 carbon atoms and at least 4 and up to 6 double bonds. Also these molecules are very well known from the art and can be obtained by standard methods of organic chemistry, for example, via transesterification of fish oils, followed by urea precipitation of the alkyl esters thus obtained, and a final extraction using non-polar solvents as described in the German patent **DE 3926658 C2** (Norsk Hydro). Fatty acids thus obtained are rich in omega-3 (all-Z)-5,8,11,14,17-eicosapentanoic acid (EPA) C 20 : 5 and (all-Z)-4,7,10,13,16,19-docosahexanoic acid (DHA) C 22 : 6. Such products can be found on the market under the trademark Omacor® (Pronova).

In a third embodiment, also linoleic acid, vaccinic acid (trans 11-octadecenoic acid) or cis-hexadecenoic acid (obtained, for example, from the plant *Thunbergia alata*) can be used.

In addition, said physiologically active fatty acid esters can not only be used in form of their lower alkyl esters or glycerides, an additional well-preferred embodiment of the present invention relates to compositions comprising esters of said fatty acids with sterols. Like glycerides, sterol esters are easily resorbed and split by the human body, however, a significant advantage comes from the fact that the cleavage of the ester bond releases a second molecule with health promoting properties. To avoid unclarities, the phrases "sterol", "stanol" and "sterin" shall be used as synonyms defining steroids showing a single hydroxyl group linked to the C-3. In addition, sterols which consist of 27 to 30 carbon atoms, may show a double bond, preferably in 5/6 position. According to the present invention, esters of CLA or omega-3 fatty acids with β-sitosterol or its hydrogenation product β-sitostanol are preferred.

### Extracts of Glycyrrhiza glabra and its active principles

The licorice root contains glycyrrhizin which is 50 times sweeter than sucrose, which encourages the production of hormones such as hydrocortisone. The extracts show an antiinflammatory action and also stimulate the adrenal cortex after steroid therapy. Main component of the extracts of *Glycyrrhiza glabra* is glycyrrhizinic acid:

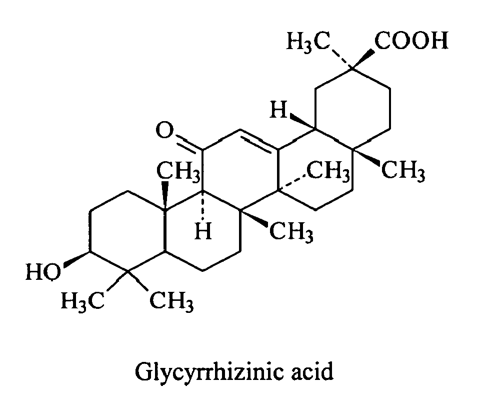

Beside the acid, also their salts, mainly the zinc salts, as well as their esters (e.g., with fatty alcohols or sterols) can be employed.

### Active compositions

The compositions according to the present invention - either component (a) alone or mixtures of components (a) and (b) - may be administered either topically or orally. Mixtures can comprise component (a) and component (b) in a weight ratio of from 99 : 1 to 50 : 50 and more particularly from 95 : 10 to 75 : 25. The highest synergistic effects, however, are observed at ratios from 92 : 8 to 80 : 20. In general, the compositions can be used in a concentration of up to about 10, particularly 0.5 to 8 and more particularly 1 to 2 % b.w. - based on the final composition. One percent, however, has been found to be particularly suitable.

In order to support the anti-itching effect and provide additional benefits to the skin, the compositions may comprise further plant extracts or their active principles, for example, chosen from the plants selected from the group consisting of *Ginkgo biloba, Oleacea europensis, Castanea sativa, Vaccinium myrtillus, Trifolium pratense, Litchi sinensis, Vitis, vinifera, Brassica oleracea, Punica granatum, Petroselinium crispum, Centella asiatica, Passiflora incarnata, Medicago sativa, Valeriana officinalis, Salix alba*, and *Harpagophytum procum-bens*.

### Macro- or micro-encapsulation

In a special embodiment of the present invention, said compositions are macro- or micro-encapsulated. "Microcapsules" are understood to be spherical aggregates with a diameter from about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("micro-sponge") and, as microparticles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third, etc., membrane. The membrane may consist of natural, semisynthetic or synthetic materials. Natural membrane materials are, for example, gum arabic, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolysates, sucrose, and waxes. Semisynthetic membrane materials are, inter alia, chemically modified celluloses, more particularly cellulose esters and ethers, for example, cellulose acetate, ethyl cellulose, hydroxypropyl cellulose, hydroxypropyl methyl cellulose, and carboxymethyl cellulose, and starch derivatives, more particularly starch ethers and esters. Synthetic membrane materials are, for example, polymers, such as polyacrylates, polyamides, polyvinyl alcohol or polyvinyl pyrrolidone. Examples of known microcapsules are the following commercial products (the membrane material is shown in brackets) *Hallcrest Microcapsules* (gelatin, gum arabic), *Coletica Thalaspheres* (maritime collagen), *Lipotec Millicapseln* (alginic acid, agar agar), *Induchem Unispheres* (lactose, microcrystalline cellulose, hydroxypropylmethyl cellulose), *Unicerin C30* (lactose, microcrystalline cellulose, hydroxypropylmethyl cellulose), *Kobo Glycospheres* (modified starch, fatty acid esters, phospholipids), *Softspheres* (modified agar agar), *Kuhs Probiol Nanospheres* (phospholipids), and *Primaspheres* or *Primasponges* (chitosan, anionic polymers). The encapsulation of the compositions according to the present invention is preferred in case the active should be liberated under controlled release. Therefore, a person skilled in the art can easily select the adequate encapsulation system by comparing the stability of the capsules under the pH conditions of the respective part of the intestine. Nevertheless, a very well working procedure has been found to use a combination of proteins like gelatin and anionic polymers, e.g., carboxy methyl cellulose. For example, a first aqueous solution of gelatin and a second aqueous solution of CMC and the active ingredients are mixed under vigorous stirring at a temperature above the melting point of the protein. Once the mixture has been allowed to cool down, the coacervation of the micro-capsules takes place indicated by an increasing turbidity of the solution. For hardening of the capsules it is suggested to reticulate the shells by adding a cross-linking agent, for example, glutaraldehyde. A detailed description of the process is given, for example, in EP 0937496 A2 (Unilever). Other suitable encapsulation methods based on gel formation agents, anionic polymers and chitosan have been described, for example, in **WO 01/01926, WO 01/01927, WO 01/01928, WO 01/01929** (Primacare), all of them being incorporated by reference.

### Examples

### Example 1

Two solutions, one containing 2.8 % b.w. gelatin (Geltec® SG-730 N, Extraco) and one containing 1.2 % b.w. sodium carboxymethylcellulose (DS-07, Aldrich, MW = 90.000) and 1.5 % b.w. conjugated linoleic acid (Tonalin® CLA, Cognis) were prepared in distilled water at 45 °C. Both solutions were adjusted to pH 6.5 using a 1 N NaOH solution. The CMC solution was added to the solution containing the gelatin, and dispersed using Ultra Turrax equipment. Thereafter, the pH was adjusted to 4.25 using a 1 N HCl solution and the mixture was cooled down to 25 °C under stirring. Subsequently, the formation of coacervates was recognized by increasing turbidity. The so-formed coacervates were hardened by adding glutaraldehyde under stirring. After a reaction time of 4 h at 25 °C, the coacervates thus obtained were filtered off using a Buchner funnel and stored at 5 °C as a slurry. The average particle size was 12.8 µm.

### Example 2

Two solutions, one containing 2.5 % b.w. gelatin (Geltec® SG-730 N, Extraco) and one containing 1.3 % b.w. sodium carboxymethylcellulose (AV 27088D, Aldrich, MW = ca. 38.000), 1.2 % b.w. conjugated linoleic acid (Tonalin® CLA, Cognis) and 0.5 % b.w. of a spray-dried extract of *Glycyrrhiza glabra* were prepared in distilled water at 45 °C. Both solutions were adjusted to pH 6.5 using a 1 N NaOH solution. The CMC solution was added to the solution containing the gelatin and dispersed using Ultra Turrax equipment. Thereafter, the pH was adjusted to 4.25 using a 1 N HCl solution and the mixture was cooled down to 25 °C under stirring. Subsequently, the formation of coacervates was recognized by increasing turbidity. The so-formed coacervates were hardened by adding glutaraldehyde under stirring. After a reaction time of 4 h at 25 °C, the coacervates thus obtained were filtered off using a Buchner funnel and stored at 5 °C as a slurry. The average particle size was 45.5 µm.

### Example 3

Two solutions, one containing 2.5 % b.w. gelatin (Geltec® SG-730 N, Extraco) and one containing 1.3 % b.w. sodium carboxymethylcellulose (AV 27088D, Aldrich, MW = ca. 38.000), 1.2 % b.w. conjugated linoleic acid (Tonalin® CLA, Cognis) and 0.5 % b.w. of a spray-dried extract of a zinc salt of glycyrrhizinic acid (Plantactiv® GLA, Cognis) were prepared in distilled water at 45 °C. Both solutions were adjusted to pH 6.5 using a 1 N NaOH solution. The CMC solution was added to the solution containing the gelatin and dispersed using Ultra Turrax equipment. Thereafter, the pH was adjusted to 4.25 using a 1 N HCl solution and the mixture was cooled down to 25 °C under stirring. Subsequently, the formation of coacervates was recognized by increasing turbidity. The so-formed coacervates were hardened by adding glutaraldehyde under stirring. After a reaction time of 4 h at 25 °C, the coacervates thus obtained were filtered off using a Buchner funnel and stored at 5 °C as a slurry. The average particle size was 45.5 µm.

### Example 4

Two solutions, one containing 2.8 % b.w. gelatin (Geltec® SG-730 N, Extraco) and one containing 1.2 % b.w. sodium carboxymethylcellulose (DS-07, Aldrich, MW = 90.000) and 1.5 % b.w. ω-unsaturated fish fatty acid (Omacor®, Pronova) were prepared in distilled water at 45 °C. Both solutions were adjusted to pH 6.5 using a 1 N NaOH solution. The CMC solution was added to the solution containing the gelatin and dispersed using Ultra Turrax equipment. Thereafter, the pH was adjusted to 4.25 using a 1 N HCl solution, and the mixture was cooled down to 25 °C under stirring. The so-formed coacervates were hardened by adding glutaraldehyde under stirring. After a reaction time of 4 h at 25 °C the coacervates thus obtained were filtered off using a Buchner funnel and stored as a slurry at 5 °C. The average particle size was 12.5 µm.

### Example 5

Two solutions, one containing 2.8 % b.w. gelatin (Geltec® SG-730 N, Extraco) and one containing 1.2 % b.w. sodium carboxymethylcellulose (DS-07, Aldrich, MW = 90.000) and 1.5 % b.w. of a synthetic triglyceride based on conjugated linoleic acid (Tonalin CLA-TG®, Cognis), were prepared in distilled water at 45 °C. Both solutions were adjusted to pH 6.5 using a 1 N NaOH solution. The CMC solution was added to the solution containing the gelatin and dispersed using Ultra Turrax equipment. Thereafter, the pH was adjusted to 4.25 using a 1 N HCl solution and the mixture was cooled down to 25 °C under stirring. After a reaction time of 4 h at 25 °C, the coacervates thus obtained were filtered off using a Buchner funnel and stored at 5 °C as a slurry. The average particle size was 12.5 µm.

### Example 6

Two solutions, one containing 2.8 % b.w. gelatin (Geltec® SG-730 N, Extraco) and one containing 1.2 % b.w. sodium carboxymethylcellulose (DS-07, Aldrich, MW = 90.000) and 1.5 % b.w. conjugated linoleic acid sterol ester were prepared in distilled water at 45 °C. Both solutions were adjusted to pH 6.5 using a 1 N NaOH solution. The CMC solution was added to the solution containing the gelatin and dispersed using Ultra Turrax equipment. Thereafter, the pH was adjusted to 4.25 using a 1 N HCl solution, and the mixture was cooled down to 25 °C under stirring. After a reaction time of 4 h at 25 °C, the coacervates thus obtained were filtered off using a Buchner funnel and stored at 5 °C as a slurry. The average particle size was 12.5 µm.

## Claims

1. Use of physiologically active fatty acids, their salts and their esters for making a medicament for the treatment of pruritus.

2. Use of active compositions comprising
(a) physiologically active fatty acids, their salts and their esters, and
(b) extracts of *Glycyrrhiza glabra* or its active principles
for making a medicament for the treatment of pruritus.

3. Use according to claims 1 or 2, **characterised in that** said physiologically active fatty acids (component a) comprise from 18 to 26 carbon atoms and from 2 to 6 double bonds.

4. Use according to any of the claims 1 to 3, **characterised in that** component (a) represents esters of said fatty acids with glycerol or sterol.

5. Use according to any of the claims 1 to 4, **characterised in that** component (a) represents conjugated linoleic acid or omega-3 fatty acids.

6. Use according to any of the claims 2 to 5, **characterised in that** component (b) represents glycyrrhizinic acid, its salts or esters or an extract of *Glycyrrhiza glabra* rich in glycyrrhizinic acid.

7. Use according to any of the claims 2 to 6, **characterised in that** they comprise component (a) and (b) in weight ratios of from 99 : 1 to 50 : 50.

8. Use according to any of the claims 1 to 7, **characterised in that** said medicaments are macro- or micro-encapsulated.
